# EUROPEAN PATENT SPECIFICATION

(11) **EP 2 823 823 B1**
(45) Date of publication and mention of the grant of the patent: **14.02.2018**
(21) Application number: 12861427.8
(22) Date of filing: 25.12.2012
(51) Int. Cl.: A61K 38/16, A61K 38/10, A61P 29/00, A61P 19/02

(54) **POLYPEPTIDES FOR THE PREPARATION OF DRUGS FOR TREATMENT OR PREVENTION OF RHEUMATOID ARTHRITIS**
POLYPEPTIDE ZUR HERSTELLUNG VON MEDIKAMENTEN ZUR BEHANDLUNG ODER PRÄVENTION VON RHEUMATOIDER ARTHRITIS
POLYPEPTIDES POUR LA PRÉPARATION DES MÉDICAMENTS POUR LE TRAITEMENT OU LA PREVENTION DE L'ARTHRITE RHEUMATOÏDE

(30) Priority: 27.12.2011 CN 201110443067
(43) Date of publication of application: 14.01.2015
(73) Proprietor: Xu, Hanmei, Nanjing, Jiangsu 211198 (CN)
(72) Inventor: PU, Chunyan, Nanjing Jiangsu 211198 (CN); SHEN, Hong, Nanjing Jiangsu 211198 (CN); XU, Hanmei, Nanjing, Jiangsu 211198 (CN)
(74) Representative: Engelhard, Markus
(86) International application number: PCT/CN2012/087442
(87) International publication number: WO 2013/097704

(56) References cited:
- EP-A1- 2 204 378
- CN-A- 1 699 408
- CN-A- 1 699 408
- CN-A- 102 205 110
- CN-A- 102 499 980
- B. ZHU ET AL: "Site-specific modification of anti-angiogenesis peptide HM-3 by polyethylene glycol molecular weight of 20 kDa", JOURNAL OF BIOCHEMISTRY, vol. 148, no. 3, 1 September 2010 (2010-09-01), pages 341-347, XP55197298, ISSN: 0021-924X, DOI: 10.1093/jb/mvq070

## Description

### Field of the Invention

The present invention relates to the pharmaceutical field, specifically to the polypeptides used in treatment or prevention of rheumatoid arthritis.

### Background of the Invention

Rheumatoid arthritis (RA) is one of the commonest autoimmune inflammatory arthropathies and major causes for disability. It is a chronic, symmetrical multi-synovial arthritis of unknown etiology. The incidence rate of RA is about 0.5%-1.0% throughout the world and about 0.4% in China. It can attack people at any age, but the risk goes higher with the increase of age. In addition, RA is closely related to gender, and the incidence ratio between male and female is 1:3. The female at the age of 45-55 are at the highest risk. The initial symptoms of RA are progressive pain and swelling in hands and wrists, particularly the swelling at the back of wrists. Though such symptoms can be relieved with common symptomatic treatments, they tend to reappear repeatedly due to irregular or underdosed medication. With the development of the disease, progressive stiffness of joints may appear early in the morning and usually lasts for more than one hour, meanwhile, some joint dysfunctions may also appear.

As is mentioned above, the etiology and pathogenesis of rheumatoid arthritis remain unknown, and its basic pathological manifestations include vasculitis and synovitis. When RA attacks, a layer of pannus forms on the synovial membrane due to angiogenesis, which consequently results in thickening of synovial membrane, increase of exudate, release of various cytokines, cartilage destruction and bone erosion. It can also affect surrounding tissues, such as muscular compartments, ligaments, tendon sheaths and muscles, and finally affect the stability of joints and lead to joint deformation and disability. The RA vasculitis may attack other organs throughout the body and manifests itself as a systemic disease.

Currently, drugs for RA treatment can be categorized into two types: symptom-controlling drugs and disease-controlling drugs. The symptom-controlling drugs can be further divided into 4 groups: 1. NSAIDs, long regarded as first-line anti-RA agents; there are more than dozens of NSAIDs available on the Chinese market; 2. glucocorticoids, very good anti-inflammatory agents; but they cannot significantly improve the symptoms and will lead to many serious side effects if being used alone for a long time. They can be used, however, in the short term in moderate dose before the slow-onset agents take effect, and would be necessary to form combined medication with the second-line agents in pulse therapy of RA flare-ups, particularly those patients with extra-articular manifestations; 3. slow-onset, anti-rheumatic drugs, usually regarded as second-line agents and including antimalarials, sodium aurothiomalate (gold), penicillamine and sulfasalazine; they take effect considerably slowly, but have positive functions in improving the overall condition of RA patients. They are also called disease-modifying antirheumatic drugs (DMARDs); 4. immunosuppressants, including methotrexate, cyclophosphamide, azathioprine, tripterygium and sinomenine, etc.

Angiogenesis is one of the main histological characteristics of rheumatoid arthritis. It causes hyperplasia of synovial membrane and infiltration of inflammatory cells-the basis for the formation of pannus and final destruction of joints. Due to angiogenesis, newly-formed blood vessels invade the joint cartilage, which, in healthy condition, contains no blood vessels. The invasion of blood vessels leads to the erosion of cartilage, pain and eventually deformation of the whole joint. Also due to angiogenesis, the thickness of patients' synovial membrane increases. Normally, the inner layer of synovial membrane in a health people constitutes only 1-2 layers of cells, while it would increase to 4-10 layers (sometimes 20 layers) of cells when RA attacks. These increased cells are not only in great quantity, but also extremely active. They can secrete a large quantity of cytokines, signaling molecules and proteases, all of which accelerate the process of joint destruction. In addition, there are a large quantity of inflammatory cells, such as T cells, B cells and monocytes infiltrating in the synovial membrane of RA patients.

Under normal physiological conditions, angiogenesis is strictly regulated and is a necessary process particularly important for reproduction, fetal development, tissue repair and wound healing. However, it takes place under many pathological conditions, including growth and metastasis of tumors, inflammatory disorders such as RA, psoriasis, osteoarthritis, inflammatory bowel disease (IBD, including Crohn's disease and ulcerative colitis) and others.

Integrins are a type of receptors widely found on the cell surface. They can induce cell-cell adhesion as well as adhesion between cells and extracellular matrix; they can also facilitate angiogenesis by means of mediating interaction between intracellular cytoskeletal proteins and extracellular matrix molecules. Currently, at least 8 types of integrins (*α1β1, α2β1, α3β1, α6β1, α6β4, α5β1, ανβ3, ανβ5*) have been found closely related to angiogenesis, among them *ανβ3* being the most important. As integrin *ανβ3* can be expressed in many cell types and combines with a variety of ligands during multicellular activities, it is involved in angiogenesis, infiltration and metastasis of tumors as well as other physiological or pathological processes such as inflammation, wound healing and blood coagulation. Integrin *ανβ3* can also recognize the Arg-Gly-Asp (RGD) sequence in its ligands, which means that polypeptides bearing the RGD sequence can function as integrin antagonists, and the RGD sequence can be adopted as a vector, targetedly delivering therapeutic polypeptides to the endothelium of newly generated blood vessels so that those diseases involving angiogenesis can be effectively treated. The RGD-bearing, angiogenesis-inhibiting polypeptides can not only block the pathways of oxygen and nutrients to the synovial membrane by inhibiting angiogenesis, but also directly lead to degeneration of blood vessels therein. Therefore, they can inhibit hyperplasia of synovial membrane of RA patients. In short, inhibition of angiogenesis is an essential step for treatment of RA, while related studies show that the proliferation and migration of endothelial cells are two crucial mechanisms for angiogenesis.

Researches have shown that on the one hand the sequence Ile-Val-Arg-Arg-Ala-Asp-Arg-Ala- Ala-Val-Pro (Seq. No.4), the 60-70 amino acids of endostatin, presents high activity in inhibiting angiogenesis under *in vitro* conditions, even higher than endostatin itself; and on the other hand the polypeptide Ala-Cys-Asp-Cys-Arg-Gly-Asp-Cys-Phe-Cys-Gly-Gly- Gly-Gly (see Seq. No.5) can specifically combine with integrin as the RGD-4C sequence it contains is one of the most important ligands of integrin. In view of these findings, a polypeptide is constructed in the present invention through combining said sequence Ala-Cys-Asp-Cys-Arg-Gly-Asp-Cys-Phe-Cys-Gly-Gly-Gly-Gly (presenting high integrin affinity and bonding capacity due to the RGD-4C sequence it contains) to the N-terminal of said sequence Ile-Val-Arg-Arg-Ala-Asp-Arg-Ala-Ala-Val-Pro (presenting high angiogenesis-inhibiting effect). The amino acid sequence of this constructed polypeptide (namely, polypeptide I) is: Ala-Cys-Asp-Cys-Arg-Gly-Asp-Cys-Phe-Cys-Gly-Gly-Gly-Gly-Ile-Val-Arg-Arg-Ala-Asp-Arg-Ala-Ala-Val-Pro. It contains 25 amino acids in total, and presents high affinity and bonding capacity to integrin (specifically *ανβ3*) on the one hand and high angiogenesis-inhibiting effect on the other, as it simultaneously integrates the RGD-4C sequence and the angiogenesis-inhibiting sequence (Seq. No.4). The prior patent "Highly Effective Angiogenesis-inhibiting Polypeptide and the Preparation and Uses Thereof" (ZL200510040378.5) disclosed the therapeutic effect of this sequence in treatment of melanoma, however, it didn't disclose the therapeutic effect of this sequence in treatment of other diseases. On the basis of a great number of experiments, the inventor of the present invention has found out that the polypeptide I presents prominent therapeutic effect on collagen-induced arthritis (CIA) and adjuvant-induced arthritis (AIA), along with fewer side effects, less dose and lower production cost in comparison with other conventional methods. Polypeptide I disclosed in the present invention is reasonably designed, presenting high feasibility in production and high effect in treatment of rheumatoid arthritis. It enormously expands the therapeutic spectrum of this series of integrin-blockers, not only providing a new perspective for developing drugs of the same kind, but also highlighting their social benefits and great market potential.

The prior patent "Highly Effective Angiogenesis-inhibiting Polypeptide and the Preparation and Uses Thereof" (ZL 200510040378.5) also disclosed another polypeptide (namely, polypeptide II), the sequence of which is Ile-Val-Arg-Arg-Ala-Asp-Arg-Ala-Ala-Val-Pro-Gly-Gly-Gly-Gly-Arg-Gly-Asp. This polypeptide contains an integrin ligand sequence: Gly-Gly-Gly-Gly-Arg-Gly-Asp (Seq. No.6) and an angiogenesis-inhibiting sequence: Ile-Val-Arg-Arg-Ala-Asp-Arg-Ala-Ala-Val-Pro; wherein the RGD (Arg-Gly-Asp) sequence contained in said integrin ligand sequence can realize effective bonding between the whole polypeptide sequence and the integrin subtype, while said angiogenesis-inhibiting sequence can effectively inhibit the process of angiogenesis. The prior patent focused only on the effect of polypeptide II on treating melanoma, in contrast, the inventor of the present invention, on the basis of further studies on polypeptide II, found out its therapeutic effect on rheumatoid arthritis, which consequently broadens its indication range and highlights its social benefits and market potential.

In addition, in the present invention, PEG modification of polypeptide II is adopted to construct polypeptide III: mPEG-SC₂₀ₖ-Ile-Val-Arg-Arg-Ala-Asp-Arg-Ala-Ala-Val-Pro-Gly-Gly-Gly-Gly-Arg-Gly-Asp. The application of polypeptide III in preparation of drugs for angiogenesis-caused eye diseases was disclosed in the prior patent: ZL 201110128464.7; in contrast, the inventor of the present invention, on the basis of further studies on polypeptide III, found out its great therapeutic effect on rheumatoid arthritis, which consequently broadens its indication range and highlights its social benefits and market potential.

### Summary of the Invention

The invention is defined in the claims.

### Objective of the Invention

On the basis of a large number of researches, the present disclosure found out that three polypeptides, namely, polypeptide I : Ala-Cys-Asp-Cys-Arg-Gly-Asp-Cys-Phe-Cys-Gly-Gly-Gly-Gly-Ile-Val-Arg-Arg-Ala-Asp-Arg-Ala-Ala-Val-Pro (two pairs of disulfide bonds contained in the sequence are pairing in the pattern of 1-4 and 2-3), polypeptide II : Ile-Val-Arg-Arg-Ala-Asp-Arg-Ala-Ala-Val-Pro-Gly-Gly-Gly-Gly-Arg-Gly-Asp and polypeptide III: mPEG-SC₂₀ₖ-Ile-Val-Arg-Arg-Ala-Asp-Arg-Ala-Ala-Val-Pro-Gly-Gly-Gly-Gly-Arg-Gly-Asp present great effect in treatment and prevention of rheumatoid arthritis.

### Technical Solutions

The application of polypeptides in preparation of drugs for treatment or prevention of rheumatoid arthritis, wherein the respective amino acid sequences of said polypeptides are:
polypeptide I : Ala-Cys-Asp-Cys-Arg-Gly-Asp-Cys-Phe-Cys-Gly-Gly-Gly-Gly-Ile-Val-Arg-Arg-Ala-Asp-Arg-Ala-Ala-Val-Pro (AP25 for short, see Seq. No.1);
polypeptide II : Ile-Val-Arg-Arg-Ala-Asp-Arg-Ala-Ala-Val-Pro-Gly-Gly-Gly-Gly-Arg-Gly-Asp (HM-3 for short, see Seq. No.2);
polypeptide III: mPEG-SC₂₀ₖ-Ile-Val-Arg-Arg-Ala-Asp-Arg-Ala-Ala-Val-Pro-Gly-Gly-Gly-Gly-Arg-Gly-Asp (PEG-HM-3 for short, see Seq. No.3);
said polypeptide I, polypeptide II and polypeptide III are obtained by means of solid-phase synthesis or recombination of expression vectors;
said polypeptide I, polypeptide II and polypeptide III can covalently couple to an adjuvant; said adjuvant can be bovine serum albumin, human serum albumin or polyethylene glycol (PEG);
said drugs for treatment or prevention of rheumatoid arthritis contain an effective amount of salts acceptable to polypeptide I, polypeptide II and polypeptide III, or if necessary, pharmaceutically acceptable vectors or excipients;
said drugs for treatment or prevention of rheumatoid arthritis can be administered through many routes, such as hypodermic injection, intramuscular injection, intravenous injection or drip, oral administration (in the form of pills, capsules, etc.) and nasal spray.

### Beneficial Effects

1. The prior patents have disclosed that polypeptide I, polypeptide II and polypeptide III have anti-tumor effect; the present invention, on the basis of further studies on polypeptide I, polypeptide II and polypeptide III, has found out that polypeptide I, polypeptide II and polypeptide III present also great therapeutic effect on rheumatoid arthritis (RA); the anti-RA effect of polypeptide I, polypeptide II and polypeptide III has never been disclosed in any academic document or patent application. This means that the anti-RA effect of polypeptide I, polypeptide II and polypeptide III disclosed in the present invention constitutes a crucial discovery on the treatment of rheumatoid arthritis. It not only broadens the indication range of these polypeptides, but also highlights their social benefits and market potential.
2. Introduction to the pharmacological mechanism of the polypeptides

Researches have indicated that on the one hand the sequence Ile-Val-Arg-Arg-Ala-Asp-Arg-Ala-Ala-Val-Pro presents great angiogenesis-inhibiting activity, and on the other the polypeptide Ala-Cys-Asp-Cys-Arg-Gly-Asp-Cys-Phe-Cys-Gly-Gly-Gly-Gly (Seq. No.5) can specifically combine with integrin as the RGD-4C sequence it contains is one of the most important ligands of integrin. In view of these findings, a polypeptide is constructed in the present invention through combining said sequence Ala-Cys-Asp-Cys-Arg-Gly-Asp-Cys-Phe-Cys-Gly-Gly-Gly-Gly (presenting high integrin affinity and bonding capacity due to the RGD-4C sequence it contains) to the N-terminal of the sequence Ile-Val-Arg-Arg-Ala-Asp-Arg-Ala-Ala-Val-Pro (presenting high angiogenesis-inhibiting effect); the polypeptide so constructed, namely, polypeptide I , presents high affinity and bonding capacity to integrin. The amino acid sequence of this integrin-blocking polypeptide is: Ala-Cys-Asp-Cys-Arg-Gly-Asp-Cys-Phe-Cys-Gly-Gly-Gly-Gly-Ile-Val-Arg-Arg-Ala-Asp-Arg-Ala-Ala-Val-Pro. As the RGD-4C sequence it contains presents high affinity and bonding capacity to integrin (specifically *ανβ3*), it can targetedly deliver the polypeptide to integrin *ανβ3*; as it also contains an angiogenesis-inhibiting sequence (Seq. No. 4), polypeptide I can simultaneously inhibit the process of angiogenesis.

The sequence Ile-Val-Arg-Arg-Ala-Asp-Arg-Ala-Ala-Val-Pro-Gly-Gly-Gly-Gly-Arg-Gly-Asp, namely, polypeptide II, contains an integrin ligand sequence (Gly-Gly-Gly-Gly-Arg-Gly-Asp) and an angiogenesis-inhibiting sequence (Ile-Val-Arg-Arg-Ala-Asp-Arg-Ala-Ala-Val-Pro); the RGD (Arg-Gly-Asp) sequence contained within said integrin ligand sequence can realize effective bonding between the whole polypeptide sequence and the integrin subtypes, while the angiogenesis-inhibiting sequence contained in polypeptide II can effectively inhibit the process of angiogenesis. In addition, in the present invention, PEG modification of polypeptide II is adopted to construct polypeptide III: mPEG-SC₂₀ₖ-Ile-Val-Arg-Arg-Ala-Asp-Arg-Ala-Ala-Val-Pro-Gly-Gly-Gly-Gly-Arg-Gly-Asp, which extends the half-life period of said polypeptide seqnence; As the RGD sequence contained in polypeptide III can targetedly combine with integrin, it can realize specific combination between polypeptide III and the endothelium of newly generated blood vessels within the joint pannus. Meanwhile, the angiogenesis-inhibiting sequence in polypeptide III can inhibit the process of angiogenesis therein, which consequently realizes desirable effect in prevention and treatment of rheumatoid arthritis.

With a large quantity of experiments, the inventor of the present invention found out that polypeptide I, polypeptide II and polypeptide III can effectively inhibit the development of adjuvant-induced RA in rats and collagen-induced RA in DBA/1 mice. *The in vivo* experiments have proved that this series of polypeptides have prominent effect in treatment of RA; they also demonstrate such advantages as little side effect, small effective dose and low production cost. The method for preparing the integrin-blocking polypeptides disclosed in the present invention is reasonable in design and presents high feasibility; and drugs prepared with this method can be applied in prevention and treatment of rheumatoid arthritis. This enormously expands the therapeutic spectrum of these integrin blockers, which on the one hand provides a new perspective for developing anti-RA drugs of the same kind, and on the other highlights their social profits and market potential.

### Brief Description of Drawings

Fig. 1 polypeptide I in improving paw swelling of CIA (DBA/1) mice
Fig. 2 polypeptide I in improving joint swelling of CIA (DBA/1) mice
Fig. 3 polypeptide I in improving joint scoring of CIA (DBA/1) mice
Fig. 4 polypeptide I in improving primary swelling of left paws of AIA rats
Fig. 5 polypeptide I in improving secondary swelling of right paws of AIA rats
Fig. 6 polypeptide I in improving joint scoring of AIA rats
Fig. 7 polypeptide II in improving paw swelling of CIA (DBA/1) mice
Fig. 8 Polypeptide II in improving joint swelling of CIA (DBA/1) mice
Fig. 9 polypeptide II in improving joint scoring of CIA in DBA/1 mice
Fig. 10 polypeptide II in improving primary swelling of left paws of CIA in rats
Fig.11 polypeptide II in improving secondary swelling of right paws of AIA rats
Fig. 12 polypeptide II in improving joint scoring of AIA rats
Fig. 13 polypeptide III in improving paw swelling of CIA (DBA/1) mice
Fig. 14 polypeptide III in improving joint swelling of CIA (DBA/1) mice
Fig. 15 polypeptide III in improving joint scoring of CIA in DBA/1 mice
Fig. 16 polypeptide III in improving primary swelling of left paws of AIA rats
Fig. 17 polypeptide III in improving secondary swelling of right paws of AIA rats
Fig. 18 polypeptide III in improving joint scoring AIA rats

### Specific Embodiments

### Embodiment 1

The solid-phase synthesis is adopted to synthesize polypeptide I , polypeptide II and polypeptide III. The synthesized products are purified with the high performance liquid chromatography (HPLC), and then their purity is determined by reversed phase high performance liquid chromatography (RP-HPLC). The synthesis method disclosed in prior patents, namely ZL 201110194918.0 and ZL201110370529.9, is adopted in the present invention.

RESULT: RP-HPLC analysis shows that the purity of the synthesized polypeptides I, polypeptide II and polypeptide III is 96.94%, 99.30%, 96.34% respectively; the result meets the required purity standard.

### Embodiment 2

### in vivo immunoprotective effect of polypeptide I on CIA mouse models

Investigating the therapeutic effect of polypeptides disclosed in the present invention on mouse collagen-induced arthritis (CIA) by means of establishing CIA mouse models. Taking 60 specific, pathogen-free DBA/1 mice (provided by Sino-British SIPPR/BK Lab. Animal Ltd, Shanghai, China; animal production license: SCXK (Shanghai) 2008-0016) as animal subjects, randomly dividing 7- or 8-week-old male mice with body weight of 18-22g into 6 groups, namely, the normal control group, model control group, polypeptide I groups including the low-dose (0.2mg/kg), medium-dose (0.4mg/kg) and high-dose (0.8mg/kg) subgroups and the ( methotrexate 1mg/kg) positive control group. Apart from the normal control group, CIA mouse models are established for all test groups on day 0: Predissolving chicken cartilage collagen type II (cII) into 4mg/ml solution in 0.1 mol/L acetic acid, and then keeping the solution in a refrigerator at 4°C overnight. On day 0 of the experiment, sufficiently emulsifying type II collagen solution with isovolumetric complete Freund's adjuvant (CFA) containing 4 mg/ml myeobaeterium tuberculosis (strain H37Rv); anesthetizing DBA/1 mice and intradermically injecting 50µl emulsion at the tail of each mouse; on day 21 of the experiment, inducing the secondary immune response by intradermically injecting 50µl emulsion at the tail of each mouse again; the emulsion used for said secondary immune response is prepared by sufficiently emulsifying 4 mg/ml type II collagen (c II) and isovolumetric incomplete Freund's adjuvant (IFA). On day 30 of the experiment, hypodermically administering drugs for each mouse; polypeptide I is further divided into low-dose (0.2mg/kg), medium-dose (0.4mg/kg) and high-dose ((0.8mg/kg), twice a day, 10 days in succession; and mice from the positive control group are administered with methotrexate (1mg/kg), once every 5 days, three times in total; mice from the normal control group and model control group are administered only with physiological saline on daily basis, 10 days in succession. During day 21 to day 70 of the experiment, evaluating the effect of the drugs on CIA mouse models by measuring the body weight, scoring the joint change and examining the diameter of left and right hind ankles, once every 3 days. On day 70 of the experiment, killing mice with cervical dislocation.
The arthritis is evaluated in accordance with the following criteria:

### 1) joint scoring

Four legs: scoring in terms of level 0 to level 4, 5 levels in total. Specifically: 0 = no red spot or swelling; 1 = small red spot or slight swelling appeared at one of front/hind toe joints); 2 = red spot or swelling appeared at more than one front/hind toe joints; 3 =paw swelling beneath ankles; 4 =paw swelling including ankles. Four feet are scored independently, with 16 as the highest point.

Scoring are conducted during day 21 to day 70 of the experiment, once every 3 days, and recording all the data.

### 2) measuring the diameter of mouse ankles

Measuring the diameter of both left and right ankles (inside-outside) and the thickness of paws of mice with a vernier caliper before the model establishment and during day 21 to day 70 of the experiment, once every 3 days; recording all the data.

The measured data are listed in the form of mean and standard deviation (mean± SD), conducting T-test with SPSS 11.0 software for all test groups and control groups, wherein * refers to p < 0.05 and ** p < 0.01.

RESULTS: comparing the model mice with the normal mice. On day 0 of the experiment, the model mice are firstly hypodermically injected at the tail with an emulsion made by collagen and isovolumetric CFA (containing deactivated myeobaeterium tuberculosis); on day 21 of the experiment, the model mice are again hypodermically injected at the tail with an emulsion made by collagen and isovolumetric IFA; on day 27 of the experiment, swelling at paws appears on CIA mice and points for arthritis scoring start increasing; the highest degree of swelling on model mice appeared on day 45-60; in addition, the body weight of model mice stops increasing since day 35 and even slightly decreases later on. The *in vivo* immunoprotective effect of polypeptide I on CIA mouse models is shown in table 1. As is shown in the table, all doses of polypeptide I present immunoprotective effect on CIA mouse models:

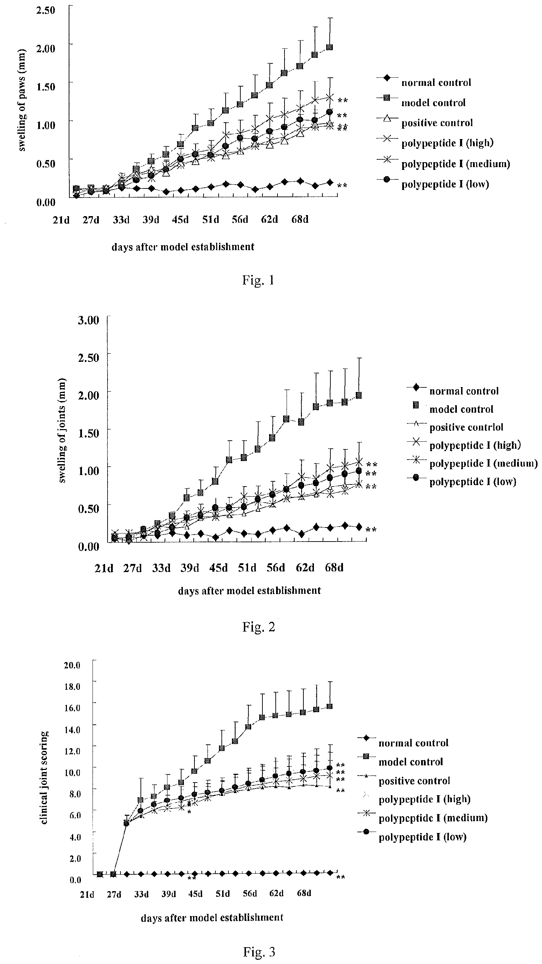

The effect of polypeptide I in improving paw swelling of CIA mouse models is demonstrated in Fig. 1. Insofar as the degree of paw swelling is concerned, both the positive control group and the polypeptide I group (including high, medium and low doses) present extremely significant difference (p <0.01) in contrast with the model control group; the test result is statistically significant. The effect of polypeptide I in improving joint swelling of CIA mouse models is demonstrated in Fig. 2. Insofar as the degree of joint swelling is concerned, both the positive control group and the polypeptide I group (including high, medium and low doses) present extremely significant difference (p <0.01) in contrast with the model control group; the test result is statistically significant. The effect of polypeptide I in improving joint scoring of CIA mouse model is demonstrated in Fig. 3. The scoring points of Polypeptide I group (including high, medium and low doses) are greatly lower than that of the control group; there exists extremely significant difference (p <0.01) between these two groups, and the test result is statistically significant.

**table 1 in vivo immunoprotective effect of polypeptide I on CIA mouse models**

| group | number (n) | dose (mg/kg) | swelling of paws (mm) | swelling of joints (mm) | clinical scoring |
|---|---|---|---|---|---|
| normal control | 10 | - | 0.18±0.05** | 0.19±0.04** | 0.00±0.00** |
| model control | 10 | - | 1.94±0.37 | 1.93±0.40 | 15.5±2.3 |
| positive control | 10 | 1 | 0.95±0.19** | 0.76±0.17** | 8.1±1.2** |
| polypeptide I(high) | 10 | 0.8 | 1.29±0.26** | 1.05±0.31** | 10.4±1.6** |
| polypeptide I (medium) | 10 | 0.4 | 0.92±0.18** | 0.76±0.17** | 9.1±1.4** |
| polypeptide I(low) | 10 | 0.2 | 1.10±0.22** | 0.93±0.23** | 9.7±1.5** |

| | | | | | |
|---|---|---|---|---|---|
| * referring to *p* < 0. 05, ** referring to *p* < 0. 01. CONCLUSION: polypeptide I has therapeutic effect on collagen-induce arthritis in mice | | | | | |

### Embodiment 3

### in vivo immunoprotective effect of polypeptide I on AIA rat models

Investigating the therapeutic effect of polypeptides disclosed in the present invention on adjuvant-induced arthritis (AIA) in rats by means of establishing AIA rat models. Taking specific, pathogen-free SD rats (provided by Sino-British SIPPR/BK Lab. Animal Ltd, Shanghai, China; animal production license: SCXK (Shanghai) 2008-0016) as animal subjects, randomly dividing 60 male rats with body weight of 140-160g into 6 groups, namely, the normal control group, model control group, polypeptide I groups including the low-dose (0.1mg/kg), medium-dose (0.2mg/kg) and high-dose (0.4 mg/kg) subgroups and the (methotrexate 1mg/kg) positive control group. Apart from the normal control group, AIA rat models are established for all test groups on day 0 by injecting at the left hind paw of all rats with 0.08ml CFA containing 10mg/ml deactivated myeobaeterium tuberculosis (strain H37RA). On day 10 of the experiment, hypodermically administering drugs for each rat; polypeptide I is further divided into low-dose (0.1mg/kg), medium-dose (0.2mg/kg) and high-dose (0.4mg/kg), twice a day, 10 days in succession; and mice from the positive control group are administered with methotrexate (1mg/kg), once every 5 days, three times in total; rats from the normal control group and model control group are administered only with physiological saline on daily basis, 10 days in succession. On day 8, 11, 14, 17, 20, 23 and 26 of the experiment, evaluating the effect of the drugs on AIA rat models by examining the diameter of both left and right hind ankles.
The arthritis is evaluated in accordance with the following criteria:

### 1) joint scoring

Four legs: scoring in terms of level 0 to level 4, 5 levels in total. Specifically: 0 = no red spot or swelling; 1 = small red spot or slight swelling appeared at one of front/hind toe joints); 2 = red spot or swelling appeared at more than one front/hind toe joints; 3 =paw swelling beneath ankles; 4 =paw swelling including ankles. Four feet are scored independently, with 16 as the highest point.

Scoring joints on day 8, 11, 14, 17, 20, 23 and 26 of the experiment and recording all the data.

### 2) measuring the diameter of rat ankles

Measuring the diameter of both left and right ankles (inside-outside) and the thickness of paws of rats with a vernier caliper before the model establishment and during day 21 to day 70 of the experiment, once every 3 days; recording all the data.

The measured data are listed in the form of mean and standard deviation (mean± SD), conducting T-test with SPSS 11.0 software for all test groups and control groups, wherein * refers to p < 0.05 and ** p < 0.01.

RESULTS: comparing the model rats with the normal rats. The primary arthritis appears at the left hind paw of model rats soon after injection of CFA containing deactivated myeobaeterium tuberculosis at the left hind paw, along with apparent swelling and ulceration; the secondary arthritis appears at the right hind paw about 10 days later, with increasingly high scores; meanwhile, apparent angiogenesis occurs at rat ears, with obvious redness and swelling; swelling also appears at tail joints. The *in vivo* immunoprotective effect of polypeptide I on AIA rat models is shown in table 2. As is shown in the table, all doses of polypeptide I present immunoprotective effect on AIA rat models:

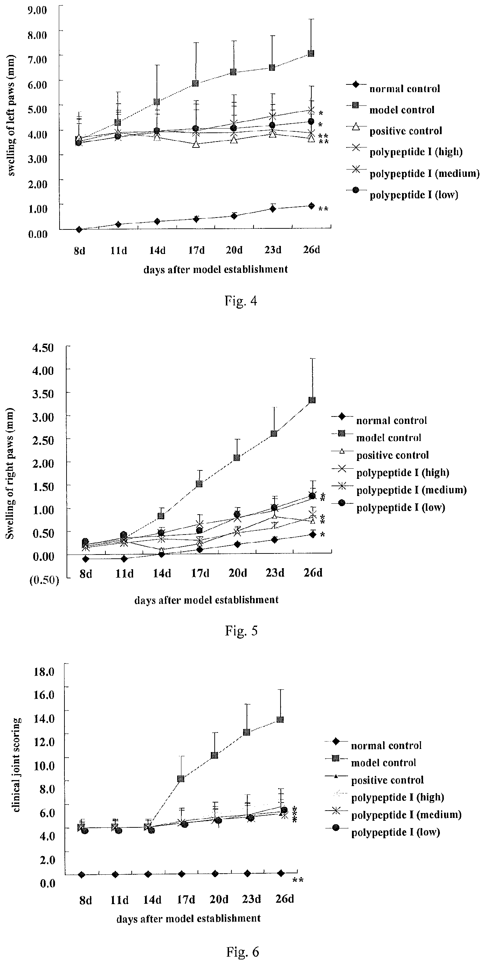

The effect of polypeptide I in improving the swelling degree of left paws induced by primary AIA is shown in Fig. 4. Insofar as the diameter of the left hind ankles is concerned, both the positive control group and polypeptide I medium-dose group present extremely significant difference (*p* <0.01) in contrast with the model control group, whereas both polypeptide I low-dose group and polypeptide I high-dose group present significant difference (*p* <0.05) in contrast with the model control group; the test result is statistically significant. The effect of polypeptide I in improving the swelling degree of right paws induced by secondary AIA is shown in Fig. 5. Insofar as the diameter of the right hind ankles is concerned, both the positive control group and the polypeptide I group (including low, medium and high doses) present significant difference (*p* <0.05) in contrast with the model control group. The effect of polypeptide I in improving joint scoring of AIA rat models is demonstrated in Fig. 6. The scoring points of polypeptide I group (including high, medium and low doses) are greatly lower than that of the control group; there exists significant difference (*p* <0.05) between these two groups, and the test result is statistically significant.

**table 2 in vivo immunoprotective effect of polypeptide I on AIA rat models**

| group | number (n) | dose (mg/kg) | swelling of left paw (mm) | swelling of right paw (mm) | clinical scoring |
|---|---|---|---|---|---|
| normal control | 10 | - | 0.90±0.18** | 0.40±0.08** | 0.0±0.0** |
| model control | 10 | - | 7.01±1.4 | 3.29±0.94 | 13.1±2.6 |
| positive control | 10 | 1 | 3.60±0.72** | 0.68±0.19** | 5.0±1.0* |
| polypeptide I(high) | 10 | 0.4 | 4.77±0.95* | 1.25±0.30* | 6.0±1.2* |
| polypeptide I(medium) | 10 | 0.2 | 3.81±0.76** | 0.79±0.18* | 5.3±1.0* |
| polypeptide I(low) | 10 | 0.1 | 4.27±0.85* | 1.18±0.31* | 5.7±1.1* |

| | | | | | |
|---|---|---|---|---|---|
| * referring to *p* < 0. 05, ** referring to *p* < 0.01. CONCLUSION: polypeptide I has therapeutic effect on adjuvant-induced arthritis in rats | | | | | |

### Embodiment 4

### in vivo immunoprotective effect of polypeptide II on CIA mouse models

The test procedure is the same as embodiment 2, only polypeptide II is used instead of polypeptide I; polypeptide II is divided into low-dose (0.8mg/kg), medium-dose (1.6mg/kg) and high-dose (3.2mg/kg) groups respectively and administered twice a day for 10 day in succession.

RESULTS: comparing the model mice with the normal mice. On day 0 of the experiment, the model mice are firstly hypodermically injected at the tail with an emulsion made by collagen and isovolumetric CFA (containing deactivated myeobaeterium tuberculosis); on day 21 of the experiment, the model mice are again hypodermically injected at the tail with an emulsion made by collagen and isovolumetric IFA; on day 27 of the experiment, swelling at paws appears on CIA mice and points for arthritis scoring start increasing; the highest degree of swelling on model mice appeared on day 45-60; in addition, the body weight of model mice stops increasing since day 35 and even slightly decreases later on. The *in vivo* immunoprotective effect of polypeptide II on CIA mouse models is shown in table 3. As is shown in the table, all doses of polypeptide II present immunoprotective effect on CIA mouse models:

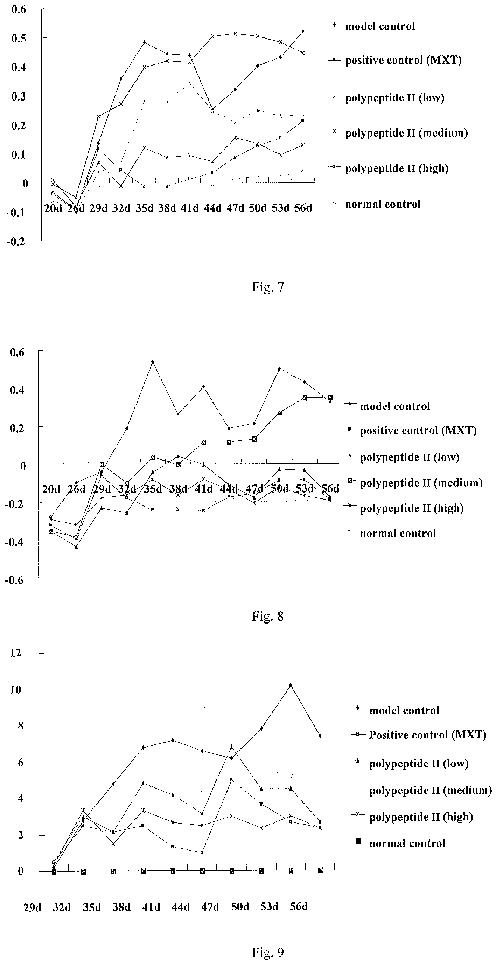

The effect of polypeptide II in improving paw swelling of CIA mouse models is demonstrated in Fig. 7. Insofar as the degree of paw swelling is concerned, both the positive control group and the polypeptide II low-dose group present extremely significant difference (p <0.01) in contrast with the model control group; the test result is statistically significant. The effect of polypeptide II in improving joint swelling of CIA mouse models is demonstrated in Fig. 8. Insofar as the degree of joint swelling is concerned, both the positive control group and the polypeptide II group (including high-dose and low-dose) present significant difference (p <0.05) in contrast with the model control group; the test result is statistically significant. The effect of polypeptide II in improving joint scoring of CIA mouse models is demonstrated in Fig. 9. The scoring points of polypeptide II group (both high and low doses) are greatly lower than that of the control group; there exists significant difference (P <0.05) between these two groups, and the test result is statistically significant.

**table 3 in vivo immunoprotective effect of polypeptide II on CIA mouse models**

| group | number (n) | dose (mg/kg) | swelling of paws (mm) | swelling of joints (mm) | clinical scoring |
|---|---|---|---|---|---|
| normal control | 10 | - | 0.04±0.07** | -0.22±0.12** | 0.00±0.00** |
| model control | 10 | - | 0.52±0.46 | 0.32±0.60 | 7.40±3.85 |
| positive control | 10 | 1 | 0.21±0.45 | -0.19±0.43* | 2.33±2.73* |
| polypeptide II (high) | 10 | 3.2 | 0.23±0.35 | -0.18±0.49* | 2.67±2.34* |
| polypeptide II (medium) | 10 | 1.6 | 0.45±0.41 | 0.35±0.80 | 5.83±2.93 |
| polypeptide II (low) | 10 | 0.8 | 0.13±0.28** | -0.19±0.28* | 2.33±3.14* |

| | | | | | |
|---|---|---|---|---|---|
| * referring to *p* < 0. 05, ** referring to *p* < 0.01. CONCLUSION: polypeptide II has therapeutic effect on collagen-induced arthritis in mice | | | | | |

### Embodiment 5

### in vivo immunoprotective effect of polypeptide II on AIA rat models

The test procedure is the same as embodiment 3, only polypeptide II is used instead of polypeptide I; polypeptide II is divided into low-dose (0.4mg/kg), medium-dose (0.8mg/kg) and high-dose (1.6 mg/kg) groups respectively and administered three times a day for 10 day in succession.

RESULTS: comparing the model rats with the normal rats. The primary arthritis appears at the left hind paw of model rats soon after injection of CFA containing deactivated myeobaeterium tuberculosis at the left hind paw, along with apparent swelling and ulceration; the secondary arthritis appears at the right high paw about 10 days later, with increasingly high scores; meanwhile, apparent angiogenesis occurs at rat ears, with obvious redness and swelling; swelling also appears at tail joints. The *in vivo* immunoprotective effect of polypeptide II on AIA rat models is shown in table 4. As is shown in the table, all doses of polypeptide II present immunoprotective effect on AIA rat models:

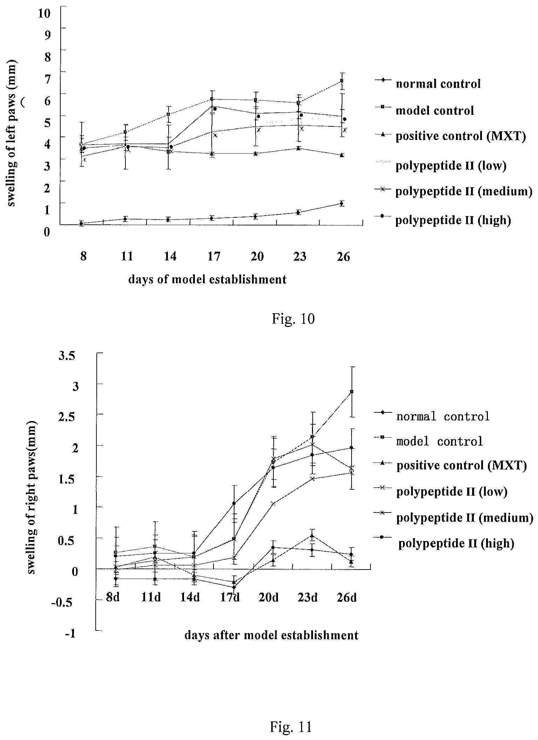

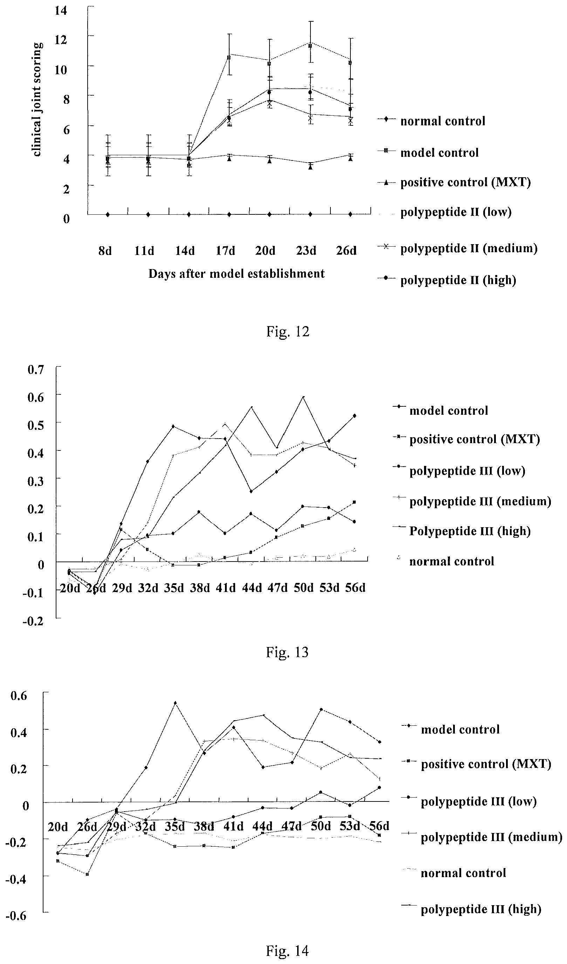

The effect of polypeptide II in improving the swelling degree of left paws induced by primary AIA is shown in Fig. 10. Insofar as the diameter of the left hind ankles is concerned, the positive control group presents extremely significant difference (p <0.01) in contrast with the model control group, whereas polypeptide II low-dose group presents significant difference (*p* <0.05) in contrast with the model control group; the test result is statistically significant. The effect of polypeptide II in improving the swelling degree of right paws induced by secondary AIA is shown in Fig. 11. Insofar as the diameter of the right hind ankles is concerned, both the positive control group and the polypeptide II lose-dose group present significant difference (*p* <0.05) in contrast with the model control group. The effect of polypeptide II in improving joint scoring of AIA rat models is demonstrated in Fig. 12. The scoring points of polypeptide II group (including medium and high doses) are lower than that of the control group; there exists significant difference (p <0.05) between these two groups, and the test result is statistically significant.

**table 4 in vivo immunoprotective effect of polypeptide II on AIA rat models**

| group | number (n) | dose (mg/kg) | swelling of left paw (mm) | swelling of right paw (mm) | clinical scoring |
|---|---|---|---|---|---|
| normal control | 10 | - | 1.00±0.47** | 0.25±0.28** | 0.00±0.00** |
| model control | 10 | - | 6.59±0.88 | 2.87±1.27 | 10.43±3.87 |
| positive control | 10 | 1 | 3.23±1.20** | 0.13±0.33** | 4.00±0.00** |
| polypeptide II (high) | 10 | 1.6 | 4.98±1.97 | 1.97±1.56 | 7.29±3.40* |
| polypeptide II (medium) | 10 | 0.8 | 4.50±1.95* | 1.63±1.57 | 6.57±4.24* |
| polypeptide II (low) | 10 | 0.4 | 5.03±1.99 | 1.56±1.66* | 8.29±4.86 |

| | | | | | |
|---|---|---|---|---|---|
| * referring to *p* < 0.05, ** referring to *p* < 0.01. CONCLUSION: polypeptide II has therapeutic effect on adjuvant-induced arthritis, and its effect is higher than that of polypeptide I. | | | | | |

### Embodiment 6

### in vivo immunoprotective effect of polypeptide III on CIA mouse models

The test procedure is the same as embodiment 2, only polypeptide III is used instead of polypeptide I; polypeptide III is divided into low-dose (10mg/kg), medium-dose (20mg/kg) and high-dose (40mg/kg) groups respectively and administered once the other day, 5 times in total.

RESULTS: comparing the model mice with the normal mice. On day 0 of the experiment, the model mice are firstly hypodermically injected at the tail with an emulsion made by collagen and isovolumetric CFA (containing deactivated myeobaeterium tuberculosis); on day 21 of the experiment, the model mice are again hypodermically injected at the tail with an emulsion made by collagen and isovolumetric IFA; on day 27 of the experiment, swelling at paws appears on CIA mice and points for arthritis scoring start increasing; the highest degree of swelling on model mice appeared on day 45-60; in addition, the body weight of model mice stops increasing since day 35 and even slightly decreases later on. The *in vivo* immunoprotective effect of polypeptide III on CIA mouse models is shown in table 5. As is shown in the table, all doses of polypeptide III present immunoprotective effect on CIA mouse models:

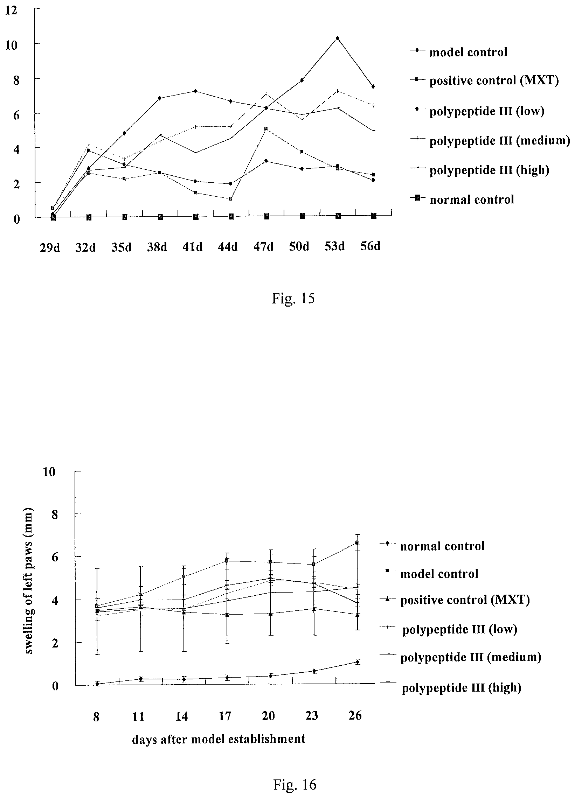

The effect of polypeptide III in improving paw swelling of CIA mouse models is demonstrated in Fig. 13. Insofar as the diameter of ankles is concerned, both the positive control group and the polypeptide III high-dose group present significant difference (*p* <0.05) in contrast with the model control group; the test result is statistically significant. The effect of polypeptide III in improving joint swelling of CIA mouse models is demonstrated in Fig. 14. Insofar as the degree of joint swelling is concerned, both the positive control group and the polypeptide III high-dose group present significant difference (*p* <0.05) in contrast with the model control group; the test result is statistically significant. The effect of polypeptide III in improving joint scoring of CIA mouse models is demonstrated in Fig. 15. The scoring points of polypeptide III high-dose group are greatly lower than that of the control group; there exists significant difference (P <0.05) between these two groups, and the test result is statistically significant.

**table 5 in vivo immunoprotective effect of polypeptide III on CIA mouse models**

| group | number (n) | dose (mg/kg) | swelling of paws (mm) | swelling of joints (mm) | clinical scoring |
|---|---|---|---|---|---|
| normal control | 10 | - | 0.04±0.07** | -0.22±0.12** | 0.00±0.00** |
| model control | 10 | - | 0.52±0.46 | 0.32±0.60 | 7.40±3.85 |
| positive control | 10 | 1 | 0.21±0.45* | -0.19±0.43* | 2.33±2.73* |
| polypeptide | 10 | 40 | 0.14±0.42* | 0.07±0.08* | 2.00±2.37* |
| III(high) | | | | | |
| polypeptide III(medium) | 10 | 20 | 0.34±0.42 | 0.12±0.55 | 6.33±4.23 |
| polypeptide III(low) | 10 | 10 | 0.37±0.35 | 0.23±0.43 | 4.83±2.99 |

| | | | | | |
|---|---|---|---|---|---|
| * referring to *p* < 0.05, ** referring to *p* < 0.01. CONCLUSION: polypeptide III has therapeutic effect on collagen-induced arthritis in mice | | | | | |

### Embodiment 7

### in vivo immunoprotective effect of polypeptide III on AIA rat models

The test procedure is the same as embodiment 3, only polypeptide III is used instead of polypeptide I; polypeptide III is divided into low-dose (10mg/kg), medium-dose (20mg/kg) and high-dose (40mg/kg) groups respectively and administered once the other day, 5 times in total.

RESULTS: comparing the model rats with the normal rats. The primary arthritis appears at the left hind paw of model rats soon after injection of CFA containing deactivated myeobaeterium tuberculosis at the left hind paw, along with apparent swelling and ulceration; the secondary arthritis appears at the right high paw about 10 days later, with increasingly high scores; meanwhile, apparent angiogenesis occurs at rat ears, with obvious redness and swelling; swelling also appears at tail joints. The *in vivo* immunoprotective effect of polypeptide III on AIA rat models is shown in table 6. As is shown in the table, all doses of polypeptide III present immunoprotective effect on AIA rat models:

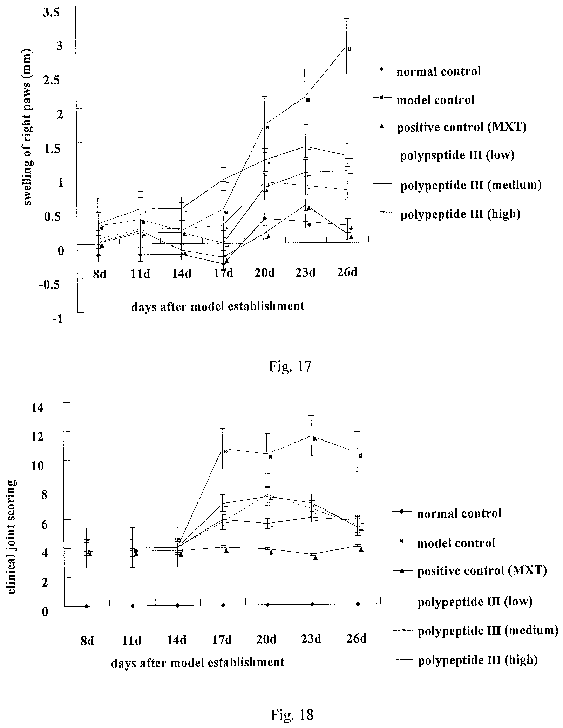

The effect of polypeptide III in improving the swelling degree of left paws induced by primary AIA is shown in Fig. 16. Insofar as the diameter of the left hind ankles is concerned, both the positive control group and polypeptide III low-dose group present extremely significant difference (*p* <0.01) in contrast with the model control group, whereas both polypeptide III medium-dose group and polypeptide III high-dose group present significant difference (*p* <0.05) in contrast with the model control group; the test result is statistically significant. The effect of polypeptide III in improving the swelling degree of right paws induced by secondary AIA is shown in Fig. 17. Insofar as the diameter of the right hind ankles is concerned, both the positive control group and polypeptide III low-dose group present extremely significant difference (*p* <0.01) in contrast with the model control group, whereas both the polypeptide III medium-dose group and the polypeptide III high-dose group present significant difference(*p* <0.05) in contrast with the model control group; the test result is statistically significant. The effect of polypeptide III in improving joint scoring of AIA rat models is demonstrated in Fig. 18. The scoring points of polypeptide III group (including low, medium and high doses) are lower than that of the control group; there exists extremely significant difference (p <0.01) between these two groups, and the test result is statistically significant.

**table 6 in vivo immunoprotective effect of polypeptide III on AIA rat models**

| group | number (n) | dose (mg/kg) | swelling of left paw (mm) | swelling of right paw (mm) | clinical scoring |
|---|---|---|---|---|---|
| normal control | 10 | - | 1.00±0.47** | 0.25±0.28** | 0.00±0.00** |
| model control | 10 | - | 6.59±0.88 | 2.87±1.27 | 10.43±3.87 |
| positive control | 10 | 1 | 3.23±1.20** | 0.13±0.33** | 4.00±0.00** |
| polypeptide III(high) | 10 | 40 | 4.40±0.91* | 1.05±1.31* | 5.29±3.90** |
| polypeptide III(medium) | 10 | 20 | 4.48±1.72* | 1.28±1.27* | 5.71±2.36** |
| polypeptide III(low) | 10 | 10 | 4.40±0.91** | 0.76±1.21** | 5.43±2.15** |

| | | | | | |
|---|---|---|---|---|---|
| * referring to *p* < 0. 05, ** referring to *p* < 0. 01. CONCLUSION: polypeptide III has therapeutic effect on adjuvant-induced arthritis, and its effect is higher than that of polypeptide I. | | | | | |

## Claims

1. A polypeptide selected from a series of polypeptides for use in a method for treatment or prevention of rheumatoid arthritis, wherein the amino acid sequence of said polypeptide is:
polypeptide III: mPEG-SC₂₀ₖ-Ile-Val-Arg-Arg-Ala-Asp-Arg-Ala-Ala-Val-Pro-Gly-Gly-Gly-Gly-Arg-Gly-Asp.

2. The polypeptide for use according to claim 1, wherein said polypeptide III is prepared by means of synthesis or recombination of expression vectors.

3. The polypeptide for use according to claim 1, wherein said polypeptide III can covalently couple to an adjuvant; said adjuvant being selected from bovine serum albumin, human serum albumin or polyethylene glycol (PEG).

4. The polypeptide for use according to claim 1, wherein said use involves the preparation of drugs for treatment or prevention of rheumatoid arthritis, wherein said drugs for treatment or prevention of rheumatoid arthritis contain an effective amount of salts acceptable to polypeptide III, or if necessary, pharmaceutically acceptable vectors or excipients.

5. The polypeptide for use according to claim 1, wherein said use involves the preparation of drugs for treatment or prevention of rheumatoid arthritis, wherein said drugs for treatment or prevention of rheumatoid arthritis can be administered through a variety of routes, including hypodermic injection, intramuscular injection, intravenous injection or drip, oral administration, e.g. in the form of pills, capsules, and nasal spray.

## Patentansprüche

1. Polypeptid, ausgewählt aus einer Serie von Polypeptiden, zur Verwendung in einem Verfahren für die Behandlung oder Prävention von rheumatoider Arthritis, wobei die Aminosäuresequenz des besagten Polypeptids ist:
Polypeptid III: mPEG-SC₂₀ₖ- Ile-Val-Arg-Arg-Ala-Asp-Arg-Ala-Ala-Val-Pro-Gly-Gly-Gly-Gly-Arg-Gly-Asp.

2. Polypeptid zur Verwendung nach Anspruch 1, wobei das Polypeptid III mittels Synthese oder Rekombination von Expressionsvektoren hergestellt ist.

3. Polypeptid zur Verwendung nach Anspruch 1, wobei das Polypeptid III kovalent an ein Adjuvans koppeln kann; wobei das Adjuvans ausgewählt ist aus bovinem Serumalbumin, humanem Serumalbumin oder Polyethylenglycol (PEG).

4. Polypeptid zur Verwendung nach Anspruch 1, wobei die Verwendung die Herstellung von Medikamenten zur Behandlung oder Prävention rheumatoider Arthritis beinhaltet, wobei die Medikamente zur Behandlung und Prävention von rheumatoider Arthritis eine wirksame Menge von für Polypeptid III akzeptablen Salzen enthalten, oder falls notwendig, pharmazeutisch akzeptable Vektoren oder Arzneihilfsstoffe.

5. Polypeptid zur Verwendung nach Anspruch 1, wobei die Verwendung die Herstellung von Medikamenten zur Behandlung oder Prävention rheumatoider Arthritis beinhaltet, wobei die Medikamente zur Behandlung oder Prävention rheumatoider Arthritis über eine Vielzahl von Verabreichungsrouten verabreicht werden kann, einschließlich hypodermaler Injektion, intramuskulärer Injektion, intravenöser Injektion oder Tropfinfusion, oraler Verabreichung, zum Beispiel in Form von Pillen, Kapseln und Nasenspray.

## Revendications

1. Polypeptide choisi dans une série de polypeptides pour une utilisation dans un procédé de traitement ou de prévention de la polyarthrite rhumatoïde, dans lequel la séquence d'acides aminés dudit polypeptide est :
polypeptide III : mPEG-SC₂₀ₖ-Ile-Val-Arg-Arg-Ala-Asp-Arg-Ala-Ala-Val-Pro-Gly-Gly-Gly-Gly-Arg-Gly-Asp.

2. Polypeptide pour une utilisation selon la revendication 1, dans lequel ledit polypeptide III est préparé au moyen d'une synthèse ou d'une recombinaison de vecteurs d'expression.

3. Polypeptide pour une utilisation selon la revendication 1, dans lequel ledit polypeptide III peut se coupler de façon covalente à un adjuvant ; ledit adjuvant étant choisi parmi la sérumalbumine bovine, la sérumalbumine humaine ou le polyéthylène glycol (PEG).

4. Polypeptide pour une utilisation selon la revendication 1, dans lequel ladite utilisation implique la préparation de médicaments pour le traitement ou la prévention de la polyarthrite rhumatoïde, dans lequel lesdits médicaments pour le traitement ou la prévention de la polyarthrite rhumatoïde contiennent une quantité efficace de sels acceptables par rapport au polypeptide III, ou si nécessaire, des vecteurs ou des excipients pharmaceutiquement acceptables.

5. Polypeptide pour une utilisation selon la revendication 1, dans lequel ladite utilisation implique la préparation de médicaments pour le traitement ou la prévention de la polyarthrite rhumatoïde, dans lequel lesdits médicaments pour le traitement ou la prévention de la polyarthrite rhumatoïde peuvent être administrés par diverses voies, y compris une injection hypodermique, une injection intramusculaire, une injection ou une perfusion intraveineuse, une administration orale, par exemple, sous la forme de pilules, de capsules, et de pulvérisation nasale.


### SEQUENCE LISTING

<110> Xu, Hanmei
<120> Polypeptides in Preparation of Drugs for Treatment or Prevention
   of Rheumatoid Arthritis
<130> X30352WOEP
<140> EP12861427.8
   <141> 2012-12-25
<150> CN20111443067
   <151> 2011-12-27
<160> 6
<170> PatentIn version 3.5
<210> 1
   <211> 25
   <212> PRT
   <213> Artificial Sequence
<220>
   <223> artificial polypeptide with high affinity to integrin and high activity in inhibiting angiogenesis
<400> 1
   Ala Cys Asp Cys Arg Gly Asp Cys Phe Cys Gly Gly Gly Gly Ile Val
   1               5                   10                  15
   Arg Arg Ala Asp Arg Ala Ala Val Pro
               20                  25
<210> 2
   <211> 18
   <212> PRT
   <213> Artificial Sequence
<220>
   <223> artificial peptide containing an integrin ligand sequence and an angiogenesis-inhibiting sequence
<400> 2
   Ile Val Arg Arg Ala Asp Arg Ala Ala Val Pro Gly Gly Gly Gly Arg
   1               5                   10                  15
   Gly Asp
<210> 3
   <211> 18
   <212> PRT
   <213> Artificial Sequence
<220>
   <223> artificial peptide obtained by PEG modification of peptide seq. id. no. 2
<220>
   <221> BINDING
   <222> (1)..(1)
   <223> artificial peptide obtained by PEG modification of peptide seq. id. no. 2 with mPEG-SC20k being attached to Ile(1)
<400> 3
   Ile Val Arg Arg Ala Asp Arg Ala Ala Val Pro Gly Gly Gly Gly Arg
   1               5                   10                  15
   Gly Asp
<210> 4
   <211> 11
   <212> PRT
   <213> Artificial Sequence
<220>
   <223> amino acid residues 60-70 of endostatin
<400> 4
   Ile Val Arg Arg Ala Asp Arg Ala Ala Val Pro
   1               5                   10
<210> 5
   <211> 14
   <212> PRT
   <213> Artificial Sequence
<220>
   <223> artificial sequence containing RGD-4C sequence
<400> 5
   Ala Cys Asp Cys Arg Gly Asp Cys Phe Cys Gly Gly Gly Gly
   1               5                   10
<210> 6
   <211> 7
   <212> PRT
   <213> Artificial Sequence
<220>
   <223> integrin ligand sequence
<400> 6
   Gly Gly Gly Gly Arg Gly Asp
   1               5